(19) Europäisches Patentamt
European Patent Office
Office européen des brevets

(11) **EP 4 552 647 A1**

(12) **EUROPEAN PATENT APPLICATION**
published in accordance with Art. 153(4) EPC

(43) Date of publication:
**14.05.2025 Bulletin 2025/20**

(21) Application number: **23834877.5**

(22) Date of filing: **05.07.2023**

(51) International Patent Classification (IPC):
**A61K 39/395** (2006.01)   **C12N 15/13** (2006.01)
**A61P 35/00** (2006.01)

(52) Cooperative Patent Classification (CPC):
**A61K 39/395; A61P 35/00; C07K 16/00**

(86) International application number:
**PCT/CN2023/105817**

(87) International publication number:
**WO 2024/008110 (11.01.2024 Gazette 2024/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.07.2022 CN 202210802103**

(71) Applicant: **Sichuan Swiftbio Biotechnology Co.,
Ltd
Chengdu, Sichuan 610000 (CN)**

(72) Inventors:
• **LIU, Dengnian
Chengdu, Sichuan 610000 (CN)**
• **WANG, Cheng
Chengdu, Sichuan 610000 (CN)**

• **MA, Xiaoli
Chengdu, Sichuan 610000 (CN)**
• **YANG, Wei
Chengdu, Sichuan 610000 (CN)**
• **WANG, Zhu
Chengdu, Sichuan 610000 (CN)**
• **LI, Wanli
Chengdu, Sichuan 610000 (CN)**

(74) Representative: **Zaboliene, Reda
Metida
Business center Vertas
Gyneju str. 16
01109 Vilnius (LT)**

Remarks:
The complete document including Reference
Table(s) and the Sequence Listing(s) can be
downloaded from the EPO website

(54) **ANTI-CCR8 ANTIBODY AND USE THEREOF**

(57) An antibody specifically binding to CCR8, an antibody fragment, an antigen-binding part thereof, and the related use. The anti-CCR8 antibody has a differentiation advantage of targeting CCR8, thereby providing a medication choice and therapeutic regimen for tumor patients.

| | BM | 76H1F11-hz | 13F3H6-hz | 13F3H6-eADCC |
|---|---|---|---|---|
| EC50 | 23.59 | 14.78 | 9.770 | 1.174 |

Fig. 4C

EP 4 552 647 A1

**Description**

**Background of the Invention**

Field of the Invention

The present disclosure relates to the field of biomedical or biopharmaceutical technology, and more specifically, to anti-CCR8 antibodies and their use.

Description of Related Art

Regulatory T cells (Tregs) are a subset of T cells that control the body's autoimmune reactivity and are closely related to the occurrence of autoimmune diseases. They also promote tumor immune evasion and accelerate tumor cell proliferation. Therefore, the development of antibody drugs targeting Tregs has become an important research direction in tumor immunotherapy.

CCR8 (C-C chemokine receptor type 8) is a G-protein-coupled receptor with a seven-transmembrane structure, consisting of 355 amino acids. It includes a short extracellular N-terminus (1-35 amino acids), and serine/threonine residues in the intracellular C-terminus serve as phosphorylation sites during receptor regulation, mediating cellular responses.

CCR8 is a chemokine receptor highly expressed on tumor-infiltrating Tregs, with low expression in Tregs of the thymus, spleen, and peripheral blood, as well as in Th2 cells. The ligand for CCR8 is chemokine CCL1, which is upregulated at sites of inflammation. CCL1 recruits FOXP3+CCR8+ Treg cells to infiltrate tumor tissues, where they exert immunosuppressive functions. Additionally, CCL1 induces upregulation of CCR8 expression on FOXP3+ Treg cells, triggers Ca2+ flux, and induces STAT3-dependent upregulation of FOXP3, CD39, IL-10, and granzyme B, enhancing the immunosuppressive activity of these tumor-infiltrating Tregs.

Studies have shown that CCR8 is significantly upregulated in tumor-infiltrating Tregs compared to Tregs in normal tissues, indicating that CCR8 is a promising therapeutic target on Tregs in the tumor microenvironment (TME) without causing systemic autoimmunity. Therefore, antibodies targeting CCR8 can clear Tregs in the TME, improve immune function in the TME, and exhibit anti-tumor potential. Currently, several pharmaceutical companies, including Bristol-Myers Squibb (BMS), are conducting clinical trials of anti-CCR8 antibodies, but all are still in Phase I clinical trials. Thus, the development of differentiated anti-CCR8 antibody drugs holds great potential for application and can provide more treatment options and regimens for cancer patients.

**Summary of the Invention**

The present disclosure develops novel anti-CCR8 antibodies or antibody fragments capable of binding to CCR8 derived from primates (human and/or monkey). It also provides anti-CCR8 antibodies, their antibody fragments, or antigen-binding portions thereof, which can be utilized as cancer therapeutics.

To achieve the above objectives, the present disclosure provides the following technical solutions:

The present disclosure provides an antibody comprising a heavy chain variable region, wherein the heavy chain variable region comprises HCDR1, HCDR2, and HCDR3, selected from any of the following combinations of amino acid sequences:

(a) SEQ ID NO: 3, 4, 5;

(b) SEQ ID NO: 6, 7, 5;

(c) SEQ ID NO: 8, 9, 5;

(d) SEQ ID NO: 10, 11, 12;

(e) SEQ ID NO: 20, 21, 22;

(f) SEQ ID NO: 23, 24, 22;

(g) SEQ ID NO: 25, 26, 22;

(h) SEQ ID NO: 27, 28, 29;

(i) SEQ ID NO: 37, 38, 39;

(j) SEQ ID NO: 40, 41, 39;

(k) SEQ ID NO: 42, 43, 39;

(l) SEQ ID NO: 44, 45, 46;

And a light chain variable region comprising LCDR1, LCDR2, and LCDR3, selected from the following combinations of amino acid sequences:

(a) SEQ ID NO: 13, 14, 15;

(b) SEQ ID NO: 16, 17, 15;

(c) SEQ ID NO: 30, 31, 32;

(d) SEQ ID NO: 33, 34, 32;

(e) SEQ ID NO: 47, 48, 49;

(f) SEQ ID NO: 50, 51, 49.

The aforementioned antibody specifically binds to one or more amino acids in the extracellular domain of CCR8, and the amino acid sequences further include derivative sequences by adding, deleting, modifying, and/or substituting one or more amino acids, while retaining the ability to bind CCR8.

Preferably, the antibody comprises HCDR1, HCDR2, HCDR3, LCDR1, LCDR2, and LCDR3, selected from the following combinations of amino acid sequences:

(a) SEQ ID NO: 37, 38, 39, 47, 48, 49;
(b) SEQ ID NO: 40, 41, 39, 47, 48, 49;
(c) SEQ ID NO: 42, 43, 49, 47, 48, 49;
(d) SEQ ID NO: 44, 45, 46, 50, 51, 49;
(e) SEQ ID NO: 3, 4, 5, 13, 14, 15;
(f) SEQ ID NO: 6, 7, 5, 13, 14, 15;
(g) SEQ ID NO: 8, 9, 5, 13, 14, 15;
(h) SEQ ID NO: 10, 11, 12, 16, 17, 15;
(i) SEQ ID NO: 20, 21, 22, 30, 31, 32;
(j) SEQ ID NO: 23, 24, 22, 30, 31, 32;
(k) SEQ ID NO: 25, 26, 22, 30, 31, 32;
(l) SEQ ID NO: 27, 28, 29, 33, 34, 32.

The aforementioned antibody specifically binds to one or more amino acids in the extracellular domain of CCR8, and the amino acid sequences further include derivative sequences by adding, deleting, modifying, and/or substituting one or more amino acids, while retaining the ability to bind CCR8.

Preferably, the aforementioned heavy chain further comprises a heavy chain constant region and/or the light chain further comprises a light chain constant region.

Preferably, the aforementioned heavy chain variable region comprises any of the amino acid sequences of SEQ ID NO: 1, 18, 35.

Preferably, the aforementioned light chain variable region comprises any of the amino acid sequences of SEQ ID NO: 2, 19, 36.

Preferably, the aforementioned antibody comprises the heavy chain variable region and the light chain variable region selected from any of the following combinations of amino acid sequences:

(a) SEQ ID NO: 1, 2;
(b) SEQ ID NO: 18, 19;
(c) SEQ ID NO: 35, 36.

More preferably, the aforementioned antibody comprises the following light chain variable region and heavy chain variable region; wherein the heavy chain variable region comprises any of the amino acid sequences of SEQ ID NO: 1, 18, 35, or is more than 95% identical to the amino acid sequences of SEQ ID NO: 1, 18, 35; the light chain variable region comprises any of the amino acid sequences of SEQ ID NO: 2, 19, 36, or is more than 95% identical to the amino acid sequences of SEQ ID NO: 2, 19, 36.

More preferably, the aforementioned antibody is capable of binding to CCR8 derived from primates, preferably humans.

More preferably, the aforementioned antibody's heavy chain variable region further comprises a human, humanized, or human framework region, and/or the antibody's light chain variable region further comprises a human, humanized, or human framework region.

More preferably, the aforementioned antibody includes monoclonal antibodies, chimeric antibodies, recombinant antibodies, fully human antibodies, humanized antibodies, bispecific antibodies, multispecific antibodies, their antibody fragments, or antigen-binding portions thereof.

More preferably, the aforementioned antibody is selected from the following combinations: (i) single-chain antibody or single-chain variable fragment, or monovalent antibody lacking a hinge region; (ii) Fab, Fab', F(ab')2; (iii) full-length antibody; (iv) antibodies comprising human IgG Fc domains.

Preferably, the aforementioned antibody has one or more of the following biological activities:

(1) Binds to both cynomolgus monkey and human CCR8;
(2) Exhibits ADCC activity;
(3) Does not bind to human CCR4;
(4) Inhibits CCL1-mediated cell migration.

In one embodiment, the present disclosure also provides a recombinant protein comprising the aforementioned antibody, antibody fragment, or antigen-binding fragment.

Preferably, the aforementioned recombinant protein further comprises a tag sequence to aid in expression and/or purification.

In another embodiment, the present disclosure also provides a nucleic acid molecule encoding the aforementioned antibody, antibody fragment, antigen-binding fragment, or recombinant protein.

In another embodiment, the present disclosure also provides a vector comprising the aforementioned nucleic acid molecule.

Preferably, the aforementioned vector includes bacterial plasmids, bacteriophages, yeast plasmids, plant cell viruses, mammalian cell viruses such as adenoviruses, lentiviruses, or retroviruses.

In another embodiment, the present disclosure also provides an engineered host cell comprising the aforementioned nucleic acid molecule or vector.

In another embodiment, the present disclosure provides an antibody-drug conjugate comprising: (i) cytotoxic agents, detection tags, drugs, cytokines, radionuclides, enzymes, or combinations thereof; and (ii) the aforementioned antibody, recombinant protein, or combinations thereof.

In a preferred embodiment, the present disclosure also provides a pharmaceutical composition comprising: (i) the aforementioned antibody, antibody fragment, antigen-binding fragment, recombinant protein, nucleic acid molecule, vector, engineered host cell, antibody-drug conjugate, or combinations thereof; and (ii) a pharmaceutically acceptable carrier.

Preferably, the aforementioned pharmaceutical composition is used to block the binding of CCR8 with any of its ligands, or used as a neutralizing antibody.

Preferably, the aforementioned pharmaceutical composition is used for depleting tumor-infiltrating Treg cells or for treating cancer.

In another embodiment, the present disclosure provides a kit or a drug delivery device comprising the aforementioned antibody, antibody fragment, antigen-binding fragment, recombinant protein, nucleic acid molecule, vector, engineered host cell, antibody-drug conjugate, or pharmaceutical composition.

Preferably, the aforementioned kit further comprises a secondary antibody against the primary antibody.

The present disclosure provides a method for producing recombinant polypeptides, the method comprising:

(i) culturing the engineered host cell under conditions suitable for expression; and
(ii) isolating the recombinant polypeptide from the culture, wherein the recombinant polypeptide is the aforementioned antibody, antigen-binding fragment, or recombinant protein.

Beneficial effects:

Compared to the prior art, the anti-CCR8 antibodies of the present disclosure have the following advantages:

(1) Strong binding affinity to primates, especially cynomolgus monkeys and human CCR8.
(2) High antibody-dependent cellular cytotoxicity (ADCC) and antibody-dependent cellular phagocytosis (ADCP) activities.
(3) No nonspecific binding to human CCR4.
(4) Ability to block CCL1-mediated cell migration with high inhibition rates.
(5) Advantageous as a method for detecting human CCR8 or as a drug for preventing or treating CCR8-related diseases in humans.

## Brief Description of the Figures

The drawings included in the specification, which form a part of this application, are provided to offer a further understanding of the present disclosure. The exemplary embodiments of the present disclosure and their descriptions are used to explain the disclosure and should not be construed as unduly limiting. In the drawings:

Figure 1A shows the cell affinity test results of the anti-human CCR8 humanized antibody with HEK293T-h.CCR8.
Figure 1B shows the cell affinity test results of the anti-human CCR8 humanized antibody with HEK293T-h.CCR8.
Figure 2 shows the cell affinity test results of the anti-human CCR8 humanized antibody with HEK293T-c.CCR8.
Figure 3A shows the binding test results of the anti-human CCR8 humanized antibody with HEK293T-h.CCR4. Count: cell number; Control: control group.
Figure 3B shows the binding test results of the anti-human CCR8 humanized antibody with HEK293T-h.CCR4. Count: cell number; Control: control group.
Figure 4A shows the cell affinity test results of the anti-human CCR8 humanized antibody with HEK293T-h.CCR8.
Figure 4B shows the ADCC activity test results of the anti-human CCR8 humanized antibody.
Figure 4C shows the ADCC activity test results of the anti-human CCR8 humanized antibody.
Figure 5 shows the ADCP activity test results of the anti-human CCR8 humanized antibody.
Figure 6 shows the test results of the anti-human CCR8 humanized antibody blocking CCL1-mediated cell migration activity.
Figure 7A shows the cell affinity test results of 13F3H6-hz with HEK293T-h.CCR8 after incubation in human plasma. Control: control group.
Figure 7B shows the cell affinity test results of 13F3H6-eADCC with HEK293T-h.CCR8 after incubation in human plasma. Control: control group.
Figure 8 shows the blood concentration test results of 13F3H6-hz and 13F3H6-eADCC in rats.
Figure 9 shows the in vivo efficacy test results of the anti-human CCR8 humanized antibody in the MC38 model of human CCR8 transgenic mice. Control: control group.
Figure 10 shows the body weight change test results in the MC38 model of human CCR8 transgenic mice treated with the anti-human CCR8 humanized antibody. Control: control group.

## Detailed Description of the Invention

The present disclosure is further described in conjunction with the accompanying drawings and specific embodiments. The scope of protection of this disclosure is not limited to the following embodiments. It should also be understood that the terms used in the embodiments of this disclosure are intended to describe specific implementations, and are not meant to limit the scope of protection of the disclosure or to serve as unique limitations. Any modifications and advantages that can be conceived by those skilled in the art without departing from the spirit and scope of the inventive concept are included within this disclosure and fall within the protection scope of the appended claims and their equivalents.

All technical and scientific terms used herein have the same meaning as commonly understood by one of ordinary skill in the art to which this disclosure belongs. In other cases, certain terms used herein will be explained in the specification. Experimental methods not specified in the following embodiments are part of the common knowledge and expertise of those skilled in the art. The features of the embodiments in this application may be combined with each other.

The following detailed description, in conjunction with the drawings, provides further understanding of the characteristics and advantages of this disclosure. The provided embodiments are merely illustrative of the methods of the disclosure and in no way limit the remainder of the content disclosed.

The antibodies of the present disclosure refer to proteins that specifically bind to primate (preferably human) CCR8, including monoclonal antibodies, chimeric antibodies, recombinant antibodies, fully human antibodies, humanized antibodies, bispecific antibodies, multispecific antibodies, antibody fragments, or antigen-binding portions thereof. Specifically, this also includes single-chain antibodies (single chain Fv), single-chain variable fragments, monovalent antibodies lacking hinge regions, disulfide-stabilized antibodies (dsFv), antigen-binding fragments (Fab), Fab', F(ab')2, full-length antibodies, and antibodies containing the human IgG Fc domain, among others.

The present disclosure also includes recombinant proteins, which are the antibodies, antibody fragments, or antigen-binding fragments of the disclosure, along with tag sequences to facilitate expression and/or purification.

The present disclosure includes nucleic acid molecules or polynucleotides encoding the antibodies of this disclosure (light chain variable region or heavy chain variable region). It also includes expression vectors containing such nucleic acid molecules, with types of vectors including bacterial plasmids, bacteriophages, yeast plasmids, plant cell viruses, and mammalian cell viruses (e.g., adenoviruses, lentiviruses, or retroviruses).

Additionally, this disclosure includes cells for expressing the disclosed antibodies, which contain the aforementioned nucleic acid molecules or vectors.

The antibodies disclosed herein can be used as drug conjugates, including cytotoxic agents, detection labels, drugs, cytokines, radionuclides, enzymes, or combinations thereof, as well as the antibodies, recombinant proteins, or combinations thereof.

The antibodies disclosed in this document can also be used as pharmaceutical compositions, particularly those that recognize human CCR8. These compositions may contain the disclosed antibodies, their fragments, or antigen-binding fragments; recombinant proteins; nucleic acid molecules; vectors; engineered host cells; antibody-drug conjugates; or combinations of the above. In addition, the compositions may include pharmaceutically acceptable carriers, such as AAV vectors. As such, the pharmaceutical compositions of this disclosure can block the binding of CCR8 to any of its ligands, and are used for the treatment and/or prevention of cancer, for removing tumor-infiltrating Treg cells, or as neutralizing antibodies.

**Abbreviations:**

CCR8: Chemokine (C-C motif) receptor 8.
CCL1: Chemokine (C-C motif) ligand 1.
Th2: T helper cell 2.
CDR: Complementarity determining region.
FR: Framework region, amino acid residues in the antibody variable region excluding CDR residues.
VH: Antibody heavy chain variable region.
VL: Antibody light chain variable region.
IgG: Immunoglobulin G.
Kabat: The immunoglobulin comparison and numbering system proposed by Elvin A. Kabat.
Chothia: The immunoglobulin numbering system proposed by Chothia et al.
IMGT: The numbering system based on the international ImMunoGeneTics information system initiated by Lefranc et al.
EC50: Half maximal effective concentration, the concentration that induces 50% of the maximal effect. ELISA: Enzyme-linked immunosorbent assay.
FACS: Fluorescence-activated cell sorting.
PCR: Polymerase chain reaction.
HRP: Horseradish peroxidase.
ADCC: Antibody-dependent cell-mediated cytotoxicity.
ADCP: Antibody-dependent cell-mediated phagocytosis.
DMEM: Dulbecco's Modified Eagle Medium.
RPMI1640: Roswell Park Memorial Institute 1640 medium.
FBS: Fetal bovine serum.

**Example 1: Construction of CCR8 Overexpression Cell Lines and Antigen Protein Expression**

The amino acid sequence of human CCR8 was referenced from the UniProt protein database (P51685), and the sequence of human CCR4 was referenced from UniProt (P51679). The amino acid sequence of cynomolgus monkey CCR8 was obtained from the NCBI protein database (XP_015300839.1). These sequences were codon-optimized by General Biosystems and synthesized into lentiviral vector pLVX or pCDNA3.4.

Viruses were prepared using a lentiviral packaging system and were used to infect HEK293T and BaF3 cells.

Stable cell lines were obtained through puromycin selection (Invivogen, Cat# ant-pr-1), including HEK293T-h.CCR8 (human), HEK293T-h.CCR4 (human), HEK293T-c.CCR8 (cynomolgus monkey), and BaF3-h.CCR8 (human). Target expression was confirmed by flow cytometry (FACS), and the resulting cell lines were used for further experiments.

The N-terminal extracellular domain of human and cynomolgus monkey CCR8 contains 35 amino acids (1-35 AA). The N-terminal extracellular domain proteins were synthesized into the pTT5-hFc or pTT5-mFc vectors. Plasmids were transiently transfected into HEK293-EBNA cells using PEImax (Polysciences, Cat# 24765-1). After seven days, the h.CCR8 (1-35 AA) and c.CCR8 (1-35 AA) antigen proteins were purified using a ProA column (GE, Mabselect XL).

**Example 2: Production of Anti-Human CCR8 Mouse Monoclonal Antibodies**

Three strains of wild-type mice (Balb/C, KM, and CD1), aged 5-6 weeks, were immunized in two batches. The first batch was immunized with the h.CCR8 (1-35 AA) protein antigen at multiple sites in the abdominal and back areas. The second batch was immunized with pCDNA3.4-h.CCR8 plasmid via tail vein injection. After four immunizations in the first batch and five in the second, serum titers were assessed using flow cytometry (FACS).

The experimental steps are as follows: Digest and centrifuge to collect HEK293T-h.CCR8, HEK293T-h.CCR4, and HEK293T-c.CCR8 cells, then wash three times with pre-cooled PBS, resuspend the cells in PBS (containing 1% BSA), and seed $3 \times 10^5$ cells per well in a 96-well V-bottom plate. Add 100-fold, 300-fold, and 900-fold diluted serum and incubate at 4°C for 1 hour. After washing three times with pre-cooled PBS, add 1 $\mu$L of anti-mouse fluorescent secondary antibody to each well and incubate at 4°C for 0.5 hours. After washing three times with pre-cooled PBS, resuspend the cells and analyze them on a flow cytometer (Beckman, model: CytoFLEX). Select mice that show high potency with HEK293T-h.CCR8 and HEK293T-c.CCR8 and low potency with HEK293T-h.CCR4 for hybridoma fusion.

Obtain the mouse spleen and lymph nodes to prepare a cell suspension, and mix it with SP2/0 mouse myeloma cells at a 1:1 ratio. Resuspend using a cell electrofusion buffer and perform electrofusion reactions using BTX-ECM2001. After the electrofusion reaction, resuspend in complete fusion medium (RPMI1640 + 20% FBS + OPI + 1×HAT) and seed 20,000-25,000 cells per well in a 96-well cell culture plate, incubating at 37°C with 5% CO2. After culturing, perform FACS screening using HEK293T-h.CCR8, HEK293T-h.CCR4, and HEK293T-c.CCR8 cells to obtain hybridoma clones with high affinity for human and cynomolgus CCR8 on the cell membrane that do not bind to human CCR4 for monoclonal selection. Cultivate the monoclonal hybridoma cells in serum-free medium (Zhuhai Kai Rui Biological, K04114), and purify the collected monoclonal supernatant using ProA (GE, Mabselect XL) resin to obtain mouse-derived antibodies.

**Example 3: Evaluation of Anti-Human CCR8 Mouse Monoclonal Antibodies**

Evaluate the affinity of anti-human CCR8 mouse monoclonal antibodies using FACS. Digest and centrifuge to collect HEK293T-h.CCR8 and HEK293T-c.CCR8 cells, then wash three times with pre-cooled PBS. Resuspend the cells in PBS (containing 1% BSA) and plate $3 \times 10^5$ cells per well in a 96-well V-bottom plate. Add gradient-diluted mouse monoclonal antibodies and incubate at 4°C for 1 hour. After washing three times with pre-cooled PBS, add 1 $\mu$L of anti-mouse fluorescent secondary antibody to each well and incubate at 4°C for 0.5 hours. After washing three times with pre-cooled PBS, resuspend the cells and analyze them using a flow cytometer (Beckman, model: CytoFLEX). Import the values into GraphPad Prism for plotting and calculate the EC50 value.

Evaluate the specificity of anti-human CCR8 mouse monoclonal antibodies using FACS. The experimental steps are as follows: digest HEK293T-h.CCR4 cells, centrifuge to collect the cells, and wash three times with pre-cooled PBS. Resuspend the cells in 1% BSA (in PBS), adding $3 \times 10^5$ cells in a volume of 50 $\mu$L to each well of a 96-well V-bottom plate. Dilute the antibody to be tested in 1% BSA (in PBS) to 20 $\mu$g/mL, take 50 $\mu$L per well, and add it to the cells, resulting in a final antibody concentration of 10 $\mu$g/mL. Mix and incubate at 4°C for 1 hour. Centrifuge to collect the cells and wash three times with pre-cooled PBS. Resuspend the cells in 50 $\mu$L of 1% BSA (in PBS), add 1 $\mu$L of fluorescent secondary antibody to each well, mix, and incubate at 4°C for 0.5 hours. Centrifuge to collect the cells, wash three times with pre-cooled PBS, resuspend the cells in 200 $\mu$L of PBS, and analyze them.

The experimental results are shown in Table 1. Select clones with high affinity for HEK293T-h.CCR8 and no non-specific binding to HEK293T-h.CCR4 for sequencing. This involves selecting clones with low $EC_{50}$ values for HEK293T-h.CCR8 and low positive rates for HEK293T-h.CCR4. Among all mouse- antibodies, only 13F3H6 showed strong binding to HEK293T-c.CCR8, with an $EC_{50}$ of 287.8 ng/mL; all other mouse antibodies showed no binding to cynomolgus CCR8 or very weak binding. The identified mouse antibody clones for sequencing are as follows: 76H1F11, 96F1C4, and 13F3H6.

**Table 1. Summary of Evaluation Results for Mouse Antibodies**

| Clone Number | Clone ID | HEK293T-h.CCR8 $EC_{50}$ (nM) | HEK293T-h.CCR4 Positive Rate (%) |
|---|---|---|---|
| 1 | 76H1F11 | 0.48 | 0.35% |
| 2 | 96F1C4 | 0.16 | 0.49% |

(continued)

| Clone Number | Clone ID | HEK293T-h.CCR8 EC$_{50}$ (nM) | HEK293T-h.CCR4 Positive Rate (%) |
|---|---|---|---|
| 3 | 13F3H6 | 0.37 | 0.23% |

**Example 4: Sequencing of Anti-Human CCR8 Mouse Monoclonal Antibodies**

The above candidate hybridoma cells, 13F3H6, 96F1C4, and 76H1F11, were collected at approximately 1×10^5 cells and underwent RNA extraction using Trizol (Invitrogen, 15596026). Subsequently, cDNA was obtained through reverse transcription using the PrimeScript RT reagent (TAKARA, RR047A) kit with PolyA. Upstream primers were designed for both the heavy chain and light chain, and downstream primers were designed for the CH1 region of the heavy chain and the CL region of the light chain. The products were amplified through PCR (Gold Mix, Tsingke BiotechnologyCo.,Ltd., TSE101) and the fragments were recovered using an agarose gel recovery kit. Samples were sent to Beijing Tsingke BiotechnologyCo., Ltd. for sequencing. The measured sequences are shown in Table 2.

**Table 2. Sequence List of Mouse Antibodies**

| Clone Number | SEQ ID No. | Heavy Chain Variable Region (VH) Sequence | SEQ ID No. | Light Chain Variable Region (VL) Sequence |
|---|---|---|---|---|
| 13F3H6 | 55 | EVQLVESGGGLVRPKGSLKFSCAA SGFTFNAYAMNWVRQAPGKGLE WVARIRTKSNNYATYYADSVKDR FTISRDDSENMLYLQMNNLKTEDT AIYYCVRHKKVREGYAMDYWGQ GTSVTVSS | 56 | DIVLTQSPASLVLSLGQRATISC RASKSVSTSGYTYMHWYQQKP GQPPKLLIYLASNLESGVPARFS GSGSGTDFTLNIHPVEEEDAAT YYCQHSRELPFTFGSGTKLEIK |
| 96F1C4 | 57 | QVQLQQSGAELVRPGASVTLSCK ASGYTFTDYEMHWVKQTPVHGLE WIGAFDPKTGGTAYNQKFKGKAIL TADKSSSTAYMELRSLTSEDSAVY YCARRRFFDVWGAGTTVTVSS | 58 | DAVMTQTPLSLPVSLGDQASIS CRSSQSLEHSNGNTYLHWYLQ KPGQSPQLLIYRVSNRFSGVLD RFSGSGSGTDFTLKISRVEAEDL GVYFCLQVTHVPFTFGTGTKLE IK |
| 76H1F11 | 59 | QVQLQQSGAELVRPGASVTLSCK ASGYTFTDYEIHWVKMTPAHGLE WIGAIKPETGGTAYNQKFKGKAIL TSDKSSSAAYMDLRSLTSEDSAVY YCTRRRYFDVWGAGTTVTVSS | 60 | DAVMTQTPLSLLVNLGDQASIS RRFSQSLEHSDGITYLHWYLQK PGQSPQLLIYRVSIRFCGVVDRF SGSGSGTDFTLKISRVEGEDLG VYFCLQVPQVPFTFGAGTKLEI K |

**Example 5: Humanization of Anti-Human CCR8 Mouse Monoclonal Antibodies**

Using the CDR grafting method, the highest homologous human germline sequence to the original mouse sequence was identified through standard BLAST analysis and used as a template. The CDRs from the mouse antibody were grafted onto the human template to construct a chimeric antibody. Next, structural analysis was conducted to identify the FR amino acids in the mouse antibody that could retain their original conformation. Corresponding amino acids in the chimeric antibody were reverted to the mouse-derived amino acids to maintain the original affinity. The constructed humanized antibody underwent computational and immunogenicity analysis to identify high immunogenicity fragments, which were then replaced with low immunogenicity fragments. The humanized sequences and the definitions of the CDRs are summarized in Table 3.

**Table 3. Humanized Antibody Sequence List**

| SEQ ID No. | Name | Sequence |
|---|---|---|
| 1 | 76H1F11-V$_H$ | QVQLVESGAEVKKPGSSVKVSCKASGYTFTDY EIHWVRQAPGQGLEWMGAIKPETGGTAYAQK FQGRVTLTSDKSTSTAYMELSSLRSEDTAVYYC TRRRYFDVWGQGTLVTVSS |
| 2 | 76H1F11-V$_L$ | DAVMTQTPLSLSVTPGQPASISCRSSQSLEHSD GITYLHWYLQKPGQSPQLLIYRVSNRFSGVPDR FSGSGSGTDFTLKISRVEAEDVGVYYCLQVTHV PFTFGQGTKVEIK |
| 3 | 76H1F11-Chothia-HCDR1 | GYTFTDY |
| 4 | 76H1F11-Chothia-HCDR2 | KPETGG |
| 5 | 76H1F11-Chothia/Abm/Kabat-HCDR3 | RRYFDV |
| 6 | 76H1F11-Abm-HCDR1 | GYTFTDYEIH |
| 7 | 76H1F11-Abm-HCDR2 | AIKPETGGTA |
| 8 | 76H1F11-Kabat-HCDR1 | DYEIH |
| 9 | 76H1F11-Kabat-HCDR2 | AIKPETGGTAYAQKFQG |
| 10 | 76H1F11-IMGT-HCDR1 | GYTFTDYE |
| 11 | 76H1F11-IMGT-HCDR2 | IKPETGGT |
| 12 | 76H1F11-IMGT-HCDR3 | TRRRYFDV |
| 13 | 76H1F11-Chothia/Abm/Kabat-LCDR1 | RSSQSLEHSDGITYLH |
| 14 | 76H1F11-Chothia/Abm/Kabat-LCDR2 | RVSNRFS |
| 15 | 76H1F11-Chothia/Abm/Kabat/IMGT-LCDR3 | LQVTHVPFT |
| 16 | 76H1F11-IMGT-LCDR1 | QSLEHSDGITY |
| 17 | 76H1F11-IMGT-LCDR2 | RVS |

(continued)

| SEQ ID No. | Name | Sequence |
|---|---|---|
| 18 | 94F1C4-V$_H$ | QVQLVESGAEVKKPGSSVKVSCKASGYTFTDYEMHWVRQAPGQGLEWMGAFDPKTGGTAYAQKFQGRVTLTADKSTSTAYMELSSLRSEDTAVYYCARRRFFDVWGQGTLVTVSS |
| 19 | 96F1C4-V$_L$ | DAVMTQTPLSLSVTPGQPASISCRSSQSLEHSNGNTYLHWYLQKPGQSPQLLIYRVSNRFSGVPDRFSGSGSGTDFTLKISRVEAEDVGVYYCLQVTHVPFTFGQGTKVEIK |
| 20 | 96F1C4-Chothia-HCDR1 | GYTFTDY |
| 21 | 96F1C4-Chothia-HCDR2 | DPKTGG |
| 22 | 96F1C4-Chothia/Abm/Kabat-HCDR3 | RRFFDV |
| 23 | 96F1C4-Abm-HCDR1 | GYTFTDYEMH |
| 24 | 96F1C4-Abm-HCDR2 | AFDPKTGGTA |
| 25 | 96F1C4-Kabat-HCDR1 | DYEMH |
| 26 | 96F1C4-Kabat-HCDR2 | AFDPKTGGTAYAQKFQG |
| 27 | 96F1C4-IMGT-HCDR1 | GYTFTDYE |
| 28 | 96F1C4-IMGT-HCDR2 | FDPKTGGT |
| 29 | 96F1C4-IMGT-HCDR3 | ARRRFFDV |
| 30 | 96F1C4-Chothia/Abm/Kabat-LCDR1 | RSSQSLEHSNGNTYLH |
| 31 | 96F1C4-Chothia/Abm/Kabat-LCDR2 | RVSNRFS |
| 32 | 96F1C4-Chothia/Abm/Kabat/IMGT-LCDR3 | LQVTHVPFT |
| 33 | 96F1C4-IMGT-LCDR1 | QSLEHSNGNTY |
| 34 | 96F1C4-IMGT-LCDR2 | RVS |
| 35 | 13F3H6-V$_H$ | EVQLVESGGGLVQPGESLRLSCAASGFTFNAYAMNWVRQAPGQGLEWVARIRTKSNNYATYYADSVKGRFTISRDDSKNTLYLEMNSLRAEDTAVYYCVRHKKVREGYAMDYWGQGTLVTVSS |
| 36 | 13F3H6-V$_L$ | DIVLTQSPASLVLSLGQRATISCRASKSVSTSGYTYMHWYQQKPGQPPKLLIYLASNLESGVPARFSGSGSGTDFTLNIHPVEEEDAATYYCQHSRELPFTFGSGTKLEIK |
| 37 | 13F3H6-Chothia-HCDR1 | GFTFNAY |
| 38 | 13F3H6-Chothia-HCDR2 | RTKSNNYA |
| 39 | 13F3H6-Chothia/Abm/Kabat-HCDR3 | HKKVREGYAMDY |
| 40 | 13F3H6-Abm-HCDR1 | GFTFNAYAMN |

(continued)

| SEQ ID No. | Name | Sequence |
|---|---|---|
| 41 | 13F3H6-Abm-HCDR2 | RIRTKSNNYATY |
| 42 | 13F3H6-Kabat-HCDR1 | AYAMN |
| 43 | 13F3H6-Kabat-HCDR2 | RIRTKSNNYATYYADSVKG |
| 44 | 13F3H6-IMGT-HCDR1 | GFTFNAYA |
| 45 | 13F3H6-IMGT-HCDR2 | IRTKSNNYAT |
| 46 | 13F3H6-IMGT-HCDR3 | VRHKKVREGYAMDY |
| 47 | 13F3H6-Chothia/Abm/Kabat-LCDR1 | RASKSVSTSGYTYMH |
| 48 | 13F3H6-Chothia/Abm/Kabat-LCDR2 | LASNLES |
| 49 | 13F3H6-Chothia/Abm/Kabat/IMGT-LCDR3 | QHSRELPFT |
| 50 | 13F3H6-IMGT-LCDR1 | KSVSTSGYTY |
| 51 | 13F3H6-IMGT-LCDR2 | LAS |
| 52 | **Kappa CL** | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 53 | **IgG1 CH** | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID No. | Name | Sequence |
|---|---|---|
| 54 | IgG1 CH eADCC | ASTKGPSVFPLAPSSKSTSGGTAALGCLVKDYF PEPVTVSWNSGALTSGVHTFPAVLQSSGLYSLS SVVTVPSSSLGTQTYICNVNHKPSNTKVDKKVE PKSCDKTHTCPPCPAPELVGGPSVFLLPPKPKD TLMISRTPEVTCVVVDVSHEDPEVKFNWYVDG VEVHNAKTKPPEEQYNSTLRVVSVLTVLHQD WLNGKEYKCKVSNKALPAPIEKTISKAKGQPR EPQVYTLPPSREEMTKNQVSLTCLVKGFYPSDI AVEWESNGQPENNYKTTPLVLDSDGSFFLYSK LTVDKSRWQQGNVFSCSVMHEALHNHYTQKS LSLSPGK |

### Example 6: Preparation of Humanized Monoclonal Antibodies Against Human CCR8

The heavy chain variable regions and light chain variable regions of the humanized monoclonal antibodies 96F1C4-hz, 13F3H6-hz, and 76H1F11-hz, along with the variable region amino acid sequence of the control antibody (BM sequence from patent CN 113260381A), were submitted to General Biologics for gene synthesis. After codon optimization, these sequences were constructed into the pTT5-h. IgG1 vector (light chain constant region SEQ NO: 52, heavy chain constant region SEQ NO: 53). The 13F3H6-hz-eADCC is an enhanced version of the 13F3H6-hz antibody with augmented antibody-dependent cell-mediated cytotoxicity (ADCC). This variant includes five amino acid mutations (F243L, R292P, Y300L, L235V, P396L) at the Fc region of the wild-type IgG1 (SEQ ID NO: 53) to generate a candidate antibody with stronger ADCC activity (SEQ ID NO: 54). After plasmid synthesis, the plasmids were extracted from bacterial cultures, and HEK293E cells were transiently transfected with PEImax. Following approximately seven days of expression, the supernatant was collected by centrifugation. The expressed supernatant was purified using MabSelectSure affinity resin. The purified antibodies were concentrated by ultrafiltration into PBS buffer, their concentrations were measured, and they were aliquoted and stored at -20°C.

### Table 4. Correspondence Table of CDRs, Variable Regions, and Constant Regions of Humanized Antibodies

| Antibody Name | $V_H$ or $V_L$ (SEQ ID No.) | Definition Method | CDR1 (SEQ ID No.) | CDR2 (SEQ ID No.) | CDR3 (SEQ ID No.) | Heavy Chain or Light Chain Constant Region (SEQ ID No.) |
|---|---|---|---|---|---|---|
| 13F3H6-HZ | 13F3H6-$V_H$ (35) | Chothia | 37 | 38 | 39 | 53 |
| | | Abm | 40 | 41 | 39 | |
| | | Kabat | 42 | 43 | 39 | |
| | | IMGT | 44 | 45 | 46 | |
| | 13F3H6-$V_L$ (36) | Chothia | 47 | 48 | 49 | 52 |
| | | Abm | 47 | 48 | 49 | |
| | | Kabat | 47 | 48 | 49 | |
| | | IMGT | 50 | 51 | 49 | |

(continued)

| Antibody Name | V_H or V_L (SEQ ID No.) | Definition Method | CDR1 (SEQ ID No.) | CDR2 (SEQ ID No.) | CDR3 (SEQ ID No.) | Heavy Chain or Light Chain Constant Region (SEQ ID No.) |
|---|---|---|---|---|---|---|
| 13F3H6-HZ-eADCC | 13F3H6-V_H (35) | Chothia | 37 | 38 | 39 | 54 |
| | | Abm | 40 | 41 | 39 | |
| | | Kabat | 42 | 43 | 39 | |
| | | IMGT | 44 | 45 | 46 | |
| | 13F3H6-V_L (36) | Chothia | 47 | 48 | 49 | 52 |
| | | Abm | 47 | 48 | 49 | |
| | | Kabat | 47 | 48 | 49 | |
| | | IMGT | 50 | 51 | 49 | |
| 76H1F11-HZ | 76H1F11-V_H (1) | Chothia | 3 | 4 | 5 | 53 |
| | | Abm | 6 | 7 | 5 | |
| | | Kabat | 8 | 9 | 5 | |
| | | IMGT | 10 | 11 | 12 | |
| | 76H1F11-V_L (2) | Chothia | 13 | 14 | 15 | 52 |
| | | Abm | 13 | 14 | 15 | |
| | | Kabat | 13 | 14 | 15 | |
| | | IMGT | 16 | 17 | 15 | |
| 96F1C4-HZ | 96F1C4-V_H (18) | Chothia | 20 | 21 | 22 | 53 |
| | | Abm | 23 | 24 | 22 | |
| | | Kabat | 25 | 26 | 22 | |
| | | IMGT | 27 | 28 | 29 | |
| | 96F1C4-V_L (19) | Chothia | 30 | 31 | 32 | 52 |

## Example 7: Evaluation of Affinity of Humanized Monoclonal Antibodies Against Human CCR8

The affinity of humanized monoclonal antibodies against human CCR8 was assessed using FACS. The experimental procedure was as follows: HEK293T-h.CCR8 cells were digested and collected by centrifugation, 5 followed by three washes with pre-cooled PBS. The cells were resuspended in 1% BSA (in PBS), with $3 \times 10^5$ cells added to each well of a 96-well V-bottom plate (50 $\mu$L per well). Prepare the antibody dilution series in 1% BSA (in PBS), starting from 40 $\mu$g/mL. Perform a 3-fold serial dilution across 10 concentration points. Add 50 $\mu$L per well to the cells, so that the final antibody concentration in the wells starts from 20 $\mu$g/mL. Mix thoroughly and incubate at 4°C for 1 hour. The cells were collected by centrifugation, washed three times with pre-cooled PBS, resuspended in 50 $\mu$L 1% BSA (in PBS), and 1 $\mu$L of fluorescent secondary antibody (Biolegend, Catalog No.: 410708) was added to each well. The mixture was incubated at 4°C for 30 minutes, followed by three washes with pre-cooled PBS. The cells were then resuspended in 200 $\mu$L PBS for detection.

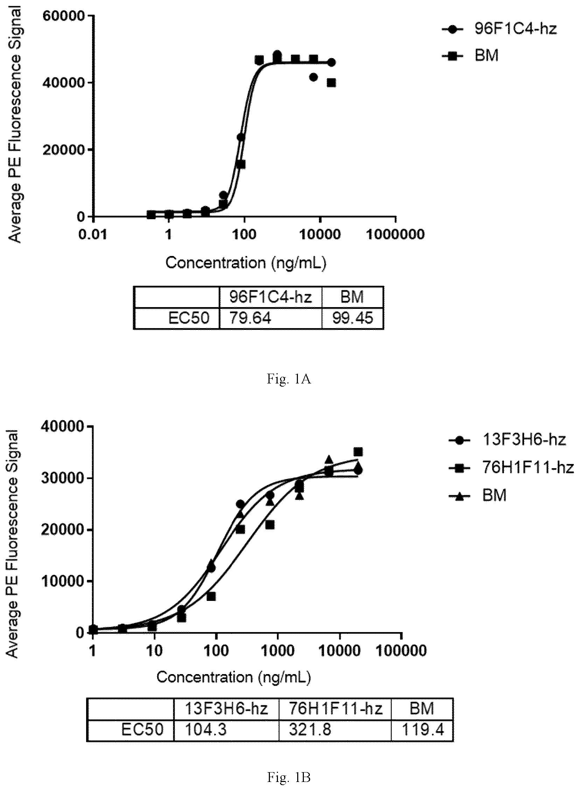

The results are shown in Figures 1A and 1B. The $EC_{50}$ values for 96F1C4-hz, 13F3H6-hz, and 76H1F11-hz against human CCR8 were 79.64 ng/mL, 104.3 ng/mL, and 321.8 ng/mL, respectively. The control antibody BM had an affinity of 109.43$\pm$9.98 ng/mL (n=2), indicating that 96F1C4-hz and 13F3H6-hz exhibited affinities comparable to the control antibody BM, while 76H1F11-hz displayed slightly weaker affinity.

## Example 8: Evaluation of Species Affinity of Humanized Monoclonal Antibodies Against CCR8 in cynomolgus

The affinity of humanized monoclonal antibodies against CCR8 in cynomolgus was measured using FACS. The procedure was similar to Example 7, with the use of HEK293T-c.CCR8 cells instead. After centrifugation and washing with pre-cooled PBS, cells were resuspended in 1% BSA (in PBS) and plated in a 96-well V-bottom plate, followed by the addition of gradient-diluted antibodies and incubation at 4°C for one hour. After washing, 1 μL of fluorescent secondary antibody (Biolegend, Catalog No.: 410708) was added and incubated again. The cells were washed and resuspended for detection using a flow cytometer (Beckman, Model: CytoFLEX).

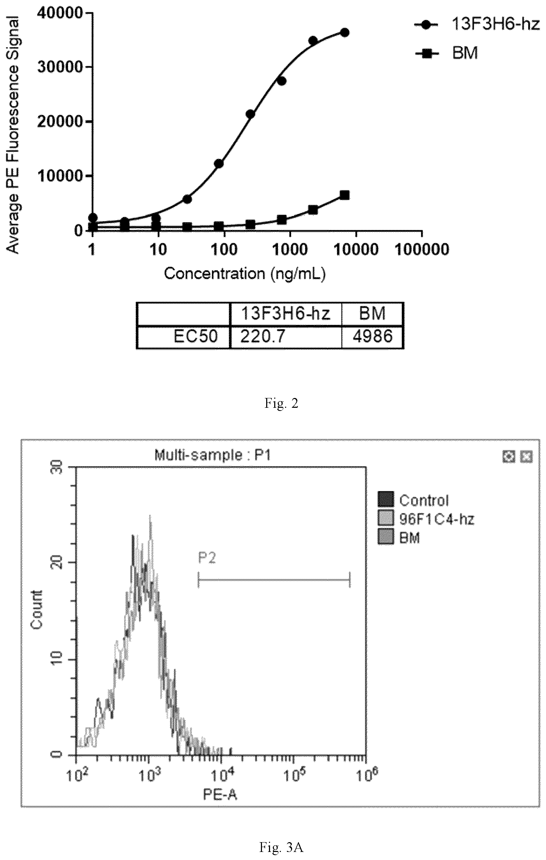

The results are shown in Figure 2. The EC50 for 13F3H6-hz binding to cynomolgus CCR8 was 220.7 ng/mL. The control antibody BM exhibited weaker binding to cynomolgus CCR8, while 96F1C4-hz and 76H1F11-hz did not bind to cynomolgus CCR8.

**Example 9: Specificity Evaluation of Humanized Monoclonal Antibodies Against Human CCR4**

The specificity of humanized monoclonal antibodies against human CCR4 was assessed using FACS. HEK293T-h.CCR4 cells were collected, washed, and resuspended in 1% BSA (in PBS). Each well of a 96-well V-bottom plate received $3\times10^5$ cells, followed by the addition of the test antibodies at a final concentration of 20 μg/mL and incubation at 4°C for one hour. After washing, 1 μL of fluorescent secondary antibody (Biolegend, Catalog No.: 410708) was added, and the mixture was incubated. The cells were washed and resuspended for detection using a flow cytometer (Beckman, Model: CytoFLEX).

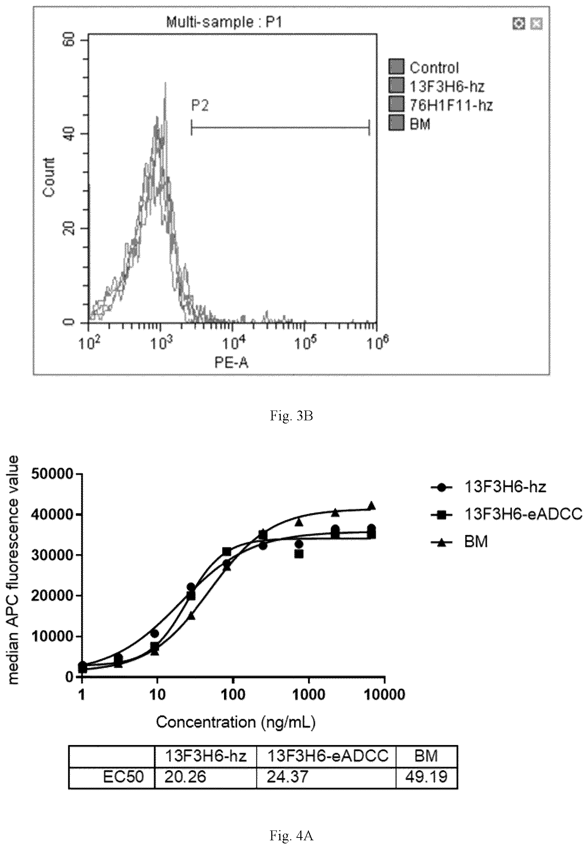

The results are shown in Figures 3A and 3B. None of the antibodies, including 96F1C4-hz, 13F3H6-hz, and 76H1F11-hz, exhibited non-specific binding to human CCR4.

**Example 10: Evaluation of Endocytosis Activity of Humanized Monoclonal Antibodies Against Human** CCR8

The endocytosis activity of humanized monoclonal antibodies against human CCR8 was assessed using FACS. HEK293T-h.CCR8 cells were collected and incubated with the test antibodies at a concentration of 10 μg/mL at 4°C for one hour. After washing, cells were resuspended in DMEM+10% FBS and split into two groups for incubation at 37°C for either 0 or 5 hours. After incubation, cells were washed and incubated with 1 μL of fluorescent secondary antibody for 30 minutes, followed by washing and resuspension for detection.

The endocytosis rate was calculated using the formula:

Endocytosis Rate (%) = [1 - (5h Sample Avg. Fluorescence - 5h Negative Control Avg. Fluorescence) / (0h Sample Avg. Fluorescence - 0h Negative Control Avg. Fluorescence] *100.

The results showed that both 96F1C4-hz and 13F3H6-hz mediated endocytosis of CCR8, with endocytosis rates of 49.57% and 51.22%, respectively. The control antibody BM had an endocytosis rate of 54.41%.

**Example 11: Evaluation of Antibody-Dependent Cell-Mediated Cytotoxicity (ADCC) of Humanized Monoclonal Antibodies Against Human CCR8**

The affinity of 13F3H6-eADCC against human CCR8 was assessed using the method described in Example 7, with the use of anti-human Alexa Fluor 647 secondary antibody (The Jackson Laboratory, Catalog No.: 109-605-088). The results are shown in Figure 4A, where 13F3H6-eADCC exhibited a high affinity for human CCR8, with an EC50 of 24.37 ng/mL. In this experimental system, 13F3H6-hz and control antibody BM exhibited affinities of 20.26 ng/mL and 49.19 ng/mL, respectively, indicating that the affinities of 13F3H6-hz, 13F3H6-eADCC, and control antibody BM were comparable.

ADCC activity was detected using a luciferase reporter system. The procedure involved collecting HEK293T-h.CCR8 cells and resuspending them in DMEM+1% FBS, followed by adding 50,000 cells per well. Jurkat-NFAT-CD16a cells were collected and resuspended in RPMI1640+1% FBS, with 100,000 cells added to each well. Gradient-diluted test antibodies were incubated with cells at 37°C for five hours. After incubation, 50 μL of Bio-Glory One-Step luciferase substrate (Suzhou Ruian, RA-GO04) was added, and the readings were taken using a microplate reader (MD, i3x).

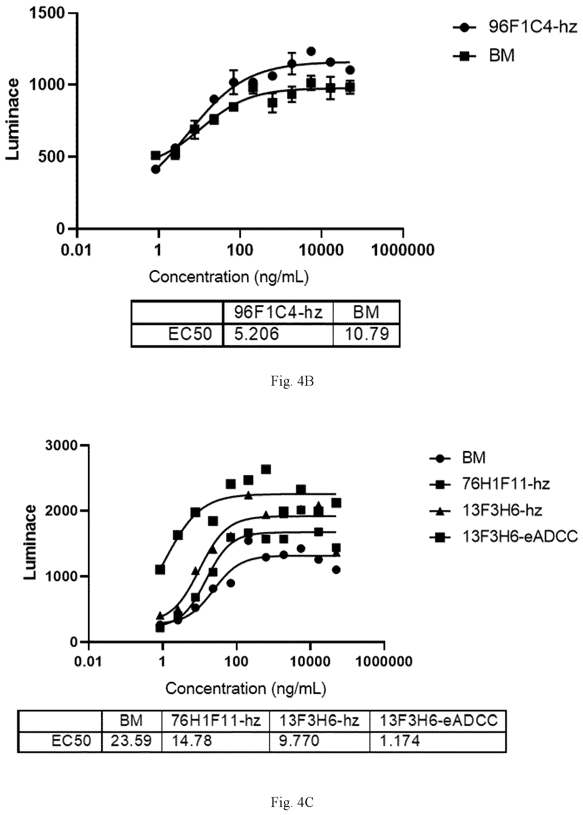

The results in Figures 4B and 4C indicate that 96F1C4-hz, 13F3H6-hz, 76H1F11-hz, and 13F3H6-eADCC exhibited strong ADCC activity, with EC50 values of 5.2 ng/mL, 9.77 ng/mL, 14.78 ng/mL, and 1.17 ng/mL, respectively. The control antibody BM had an EC50 value of 17.19±6.4 ng/mL (n=2). These results demonstrate that the ADCC activities of 96F1C4-hz, 13F3H6-hz, and 13F3H6-eADCC were superior to that of control antibody BM.

**Example 12: Evaluation of Antibody-Dependent Cell-Mediated Phagocytosis (ADCP) Activity of Humanized Monoclonal Antibodies Against Human CCR8**

The ADCP activity of humanized monoclonal antibodies against human CCR8 was assessed using a luciferase reporter system. The procedure involved collecting HEK293T-h.CCR8 cells, resuspending them in DMEM+1% FBS, and plating 50,000 cells per well. Jurkat-FcγRIIa-H131 (Vazyme Biotech Co.,Ltd, DD1304) cells were collected and resuspended in RPMI1640+1% FBS, with 100,000 cells added to each well. Gradient-diluted test antibodies were incubated with cells at 37°C for five hours. After incubation, 50 μL of Bio-Glory One-Step luciferase substrate (Suzhou Rhinobio, RA-GO04) was added, and the readings were taken using a microplate reader (MD, i3x).

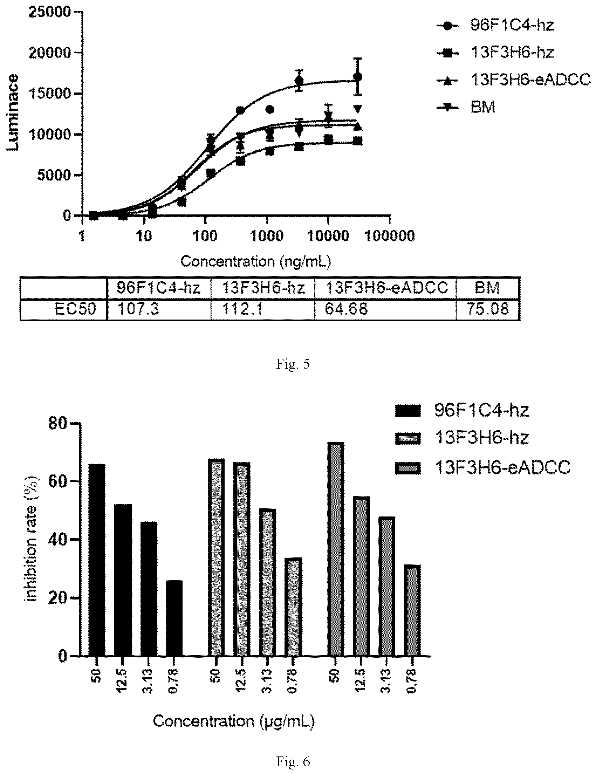

The experimental results are shown in Figure 5. 96F1C4-hz, 13F3H6-hz, and 13F3H6-eADCC all exhibit strong ADCP activity, with EC50 values of 107.3 ng/mL, 112.1 ng/mL, and 64.68 ng/mL, respectively. The EC50 value of the control antibody BM is 75.08 ng/mL. These results indicate that the ADCP activity of 96F1C4-hz, 13F3H6-hz, and 13F3H6-eADCC is comparable to that of the control antibody BM.

**Example 13: Blocking of CCL1-mediated Cell Migration by Anti-human CCR8 Humanized Monoclonal Antibody**

In this example, the blocking activity of anti-human CCR8 humanized monoclonal antibody against CCL1-mediated cell migration was tested using the Transwell method.

The experimental steps are as follows: BaF3-h.CCR8 cells were centrifuged, and collected, then resuspended in medium (RPMI1640 + 10% FBS + 1% P/S) and adjusted to a density of 800,000/mL. A total of 125 μL of the suspension was added to the upper chamber of a Transwell (Corning, Cat. No. 3421), and 125 μL of antibody diluted in medium was also added. CCL1 was diluted in medium to 5 ng/mL, and 500 μL was added to the lower chamber. The plates were incubated for 5 hours in a 37°C, 5% CO2 incubator. After incubation, the upper chamber was discarded, and 200 μL of the mixed liquid from the lower chamber was taken and added to 20 μL of CellTiter (Suzhou Rhinobio, Cat. No. RA-GL11-A). After shaking for 3 minutes, the reading was taken using a microplate reader (MD, i3x), and the inhibition rate was calculated.

The experimental results are shown in Figure 6. 96F1C4-hz, 13F3H6-hz, and 13F3H6-eADCC were all able to block CCL1-mediated cell migration, with inhibition rates at 50 μg/mL of 60.07%, 67.98%, and 73.52%, respectively, while the inhibition rate of the control antibody BM was 83.07%.

In summary, the comparative data of the examples are presented in Table 5. Compared with the control antibody BM, 13F3H6-hz, 13F3H6-hz-eADCC, and 96F1C4-hz exhibited stronger affinity for human CCR8. Among them, only 13F3H6-hz showed strong affinity for cynomolgus CCR8. The ADCC activity of the four clones, 96F1C4-hz, 76H1F11-hz, 13F3H6-hz, and 13F3H6-hz-eADCC, was higher than that of the control antibody BM, with the ADCP activity of 13F3H6-hz-eADCC being higher than that of the control antibody BM. In addition, the blocking effect of 96F1C4-hz, 76H1F11-hz, 13F3H6-hz, and 13F3H6-hz-eADCC on CCL1-mediated cell migration was comparable to that of the control antibody BM.

**Table 5. Comparison of Data between the Antibodies Disclosed in This Publication and the Control Antibody**

|  | **96F1C4-hz** | **76H1F11-hz** | **13F3H6-hz** | **13F3H6-hz-eADCC** | **Control antibody BM** |
|---|---|---|---|---|---|
| **Human CCR8 EC$_{50}$ (ng/mL) test 1** | 79.64 | 321.8 | 104.3 | - | 109.43±9.98 (n=2) |
| **Human CCR8 EC$_{50}$ (ng/mL) test 2** | - | - | 20.26 | 24.37 | 49.19 |
| **Cynomolgus CCR8 EC$_{50}$ (ng/mL)** | Does not bind to cynomolgus CCR8 | Does not bind to cynomolgus CCR8 | 220.7 | - | Weak binding |
| **Human CCR4 EC$_{50}$ (ng/mL)** | Does not bind to human CCR4 | Does not bind to human CCR4 | Does not bind to human CCR4 | - | Does not bind to human CCR4 |
| **Endocytosis rate (%)** | 49.57 | - | 51.22 | - | 54.41 |
| **ADCC activity EC$_{50}$ (ng/mL)** | 5.2 | 14.78 | 9.77 | 1.17 | 17.19±6.4 (n=2) |
| **ADCP activity EC$_{50}$ (ng/mL)** | 107.3 | - | 112.1 | 64.68 | 75.08 |
| **Inhibition rate of CCL1-mediated cell migration (%)** | 60.07 | - | 67.98 | 73.52 | 83.07 |

The above description is only a preferred embodiment of the present disclosure and is not intended to limit the disclosure. All references mentioned in this disclosure are fully cited herein for reference. Furthermore, it should be understood that after reading the above teachings of this disclosure, those skilled in the art may make various changes or modifications within the spirit and principles of this disclosure, and these equivalent modifications also fall within the scope defined by the claims of this application.

### Example 14: Accelerated Stability Testing of Humanized Anti-Human CCR8 Antibody

The accelerated stability of the humanized anti-human CCR8 antibody was assessed using the following method. The experimental steps are as follows: The humanized anti-human CCR8 antibody was ultrafiltered to PBS (pH = 7.4) at a concentration of 5 mg/mL and sterilized by filtration. It was then stored at 37°C for 0, 7, and 14 days, and subjected to 4 and 8 freeze-thaw cycles at -80°C. The purity of the samples was subsequently assessed by SEC-HPLC and CE-SDS.
The SEC-HPLC detection method is as follows: Instrument: Waters Alliance e2695 HPLC; Column: Thermo MabPac SEC-1, 5 $\mu$m, 7.8*300 mm; Mobile phase: 61 mmol/L Na2HPO4, 39 mmol/L NaH2PO4, 200 mmol/L NaCl, 5% IPA; Instrument parameters: Sample chamber temperature: 8°C; Column temperature: 30°C; Flow rate: 0.5 mL/min; Injection volume: 20 $\mu$g; Detection wavelength: 280 nm; Isocratic run: 30 min.
The CE-SDS detection method is as follows: The sample was diluted to 4 mg/mL with ultrapure water. A 25 $\mu$L aliquot was placed in a centrifuge tube, and 75 $\mu$L of a pre-mixed solution (SDS Sample buffer + iodoacetamide) was added, mixed, and centrifuged at 10,000 g for 1 minute. It was treated at 70°C for 5 minutes. Afterward, it was allowed to cool to room temperature, centrifuged again at 10,000 g for 1 minute, mixed, and transferred to an injection vial.
The analysis was performed using a SCIEX PA800 plus capillary electrophoresis instrument, with the following instrument parameters set: Capillary: 30.2 cm * 50 $\mu$m (bare), effective length: 20 cm, capillary column temperature: 25°C, sample tray temperature: 15°C, detection wavelength set at 220 nm; sampling frequency: 4 Hz.
The experimental results are shown in Table 6. Both 13F3H6-hz and 13F3H6-eADCC exhibited good stability under accelerated and repeated freeze-thaw conditions, with SEC purity above 99%, CE-SDS non-reduced purity around 90%, and CE-SDS reduced purity above 97%, indicating that 13F3H6-hz and 13F3H6-eADCC possess good accelerated stability.

### Table 6. Accelerated Stability Study of Anti-CCR8 Humanized Antibody

| Sample name | Treatment conditions | SEC | | | CE-SDS | CE-SDS |
| --- | --- | --- | --- | --- | --- | --- |
| | | Polymer (%) | Main peak (%) | Small molecule impurities (%) | Non-reduced purity (%) | Reduced purity (H+L) (%) |
| **13F3H6-hz** | 37°C, 0 day (Control) | 0 | 100 | 0 | 91.5 | 98.6 |
| | 37°C, 7 days | 0 | 100 | 0 | 89.9 | 98.4 |
| | 37°C, 14 days | 0 | 100 | 0 | 90.1 | 97.6 |
| | -80°C, 4 freeze-thaw cycles | 0 | 100 | 0 | ND | ND |
| | -80°C, 8 freeze-thaw cycles | 0 | 100 | 0 | ND | ND |
| 13F3H6-**eADCC** | 37°C, 0 day (Control) | 0 | 100 | 0 | 92.5 | 99.2 |
| | 37°C, 7天 | 0 | 100 | 0 | 91.8 | 98.4 |
| | 37°C, 14天 | 0 | 99.68 | 0.32 | 89.2 | 98.4 |
| | -80°C, 4 freeze-thaw cycles | 0 | 100 | 0 | ND | ND |
| | -80°C, 8 freeze-thaw cycles | 0 | 100 | 0 | ND | ND |
| *Note: ND indicates not detected. | | | | | | |

### Example 15: Plasma Stability Testing of Humanized Anti-Human CCR8 Antibody

Humanized candidate antibodies 13F3H6-hz and 13F3H6-eADCC were diluted to 20 $\mu$g/mL in human plasma, filtered through a 0.22 $\mu$m filter for sterilization, with each candidate set at two time points of 7 days and 14 days, 500 $\mu$L per tube. After sealing with a sealing film, the tubes were placed in a 37°C incubator for incubation, and samples were collected at 7 days and 14 days for storage at -80°C. The affinity of the samples after plasma incubation was assessed using FACS, as described in the previous examples.

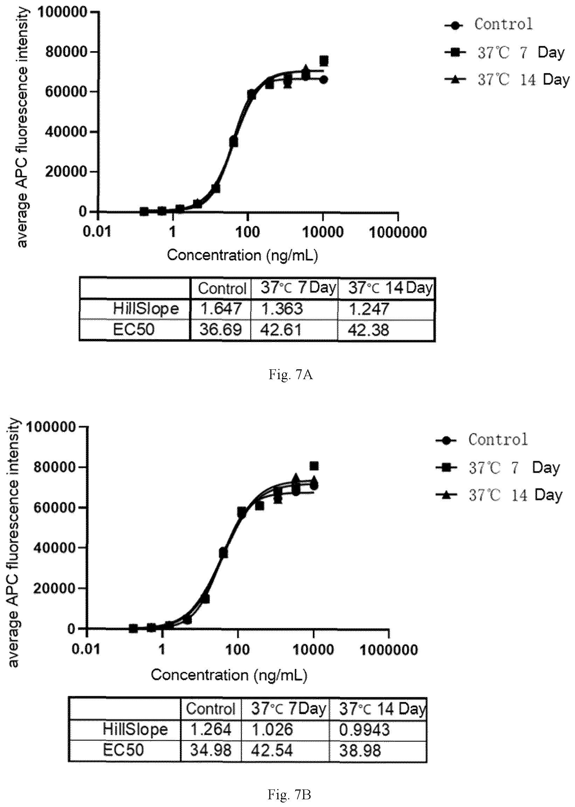

The experimental results are shown in Figures 7A and 7B. There was no significant change in the affinity of 13F3H6-hz and 13F3H6-eADCC after incubation in plasma for 7 days and 14 days, indicating that 13F3H6-hz and 13F3H6-eADCC can remain stable in human plasma.

## Example 16: Low pH Incubation Stability Testing of Humanized Anti-Human CCR8 Antibody

The 13F3H6-hz and 13F3H6-eADCC antibodies were ultrafiltered to acetate-sodium acetate buffer (pH = 3.5) and incubated at room temperature for 4 hours. After the incubation, the purity of the samples was assessed using SEC-HPLC and CE-SDS, as described in the previous examples.

The experimental results are shown in Table 7. After incubation under low pH conditions, the purity of 13F3H6-hz and 13F3H6-eADCC did not change significantly, indicating that both can maintain stability under low pH conditions.

### Table 7. Stability Study of Anti-CCR8 Humanized Antibody under Low pH Incubation

| Sample name | Treatment conditions | SEC | | | CE-SDS | CE-SDS |
|---|---|---|---|---|---|---|
| | | Polymer (%) | Main peak (%) | Small moleculeimpurities (%) | Non-reducedpurity (%) | Reduced purity(H+L) (%) |
| 13F3H6-hz | PH=7.4, 4 h (Control) | 0 | 100 | 0 | 91.5 | 98.6 |
| | PH=3.5, 4 h | 0 | 100 | 0 | 93.2 | 98.3 |
| 13F3H6-eADCC | PH=7.4, 4 h (Control) | 0 | 100 | 0 | 92.5 | 99.2 |
| | PH=3.5, 4 h | 0 | 100 | 0 | 91.8 | 98.7 |

## Example 17: Pharmacokinetic Detection of Humanized Anti-Human CCR8 Antibody in Rats

To verify the metabolism of humanized anti-human CCR8 antibodies in rats, the candidate samples 13F3H6-hz and 13F3H6-eADCC were administered via tail vein injection to male rats (Chengdu Dossy Experimental Animal Co., Ltd., China.), with 3 rats per group and a dosage of 10 mg/kg. Serum samples were collected at 1h, 2h, 6h, 24h, 48h, 96h, 168h, and 336h post-administration.

The blood concentration was measured using ELISA with the following detection method:

(1) Coating: Dilute the Human IgG-heavy and light chain monkey-adsorbed antibody (BETHYL, A80-319A) to a final concentration of 1 $\mu$g/mL in the coating solution, add 100 $\mu$L/well to the ELISA plate, cover, and incubate overnight at 2-8 °C (15-18 h).

(2) Washing: Discard the coating solution and wash 3 times with 0.025% PBST, 300 $\mu$L/well, and pat dry.

(3) Blocking: Add blocking solution (2% BSA), 300 $\mu$L/well, cover, and incubate at 37 °C for 2 h $\pm$ 20 min.

(4) Washing: Wash 3 times with 0.025% PBST, 300 $\mu$L/well, and pat dry.

(5) Sample addition: Add standard curve samples (diluted from 50 ng/mL of 13F3H6-hz and 13F3H6-eADCC in 2-fold dilutions, 8 concentration points) and test samples in duplicate, 100 $\mu$L/well, cover, and incubate at 37 °C for 1 h $\pm$ 5 min.

(6) Washing: Wash 3 times with 0.025% PBST, 300 $\mu$L/well, and pat dry.

(7) Adding secondary antibody: Dilute Goat Anti-Human IgG, Monkey ads-HRP (Southern Biotech, 2049-05) 8000 times in dilution solution (2% BSA), 100 $\mu$L/well, cover, and incubate at 37 °C for 1 h $\pm$ 5 min.

(8) Washing: Wash 3 times with 0.025% PBST, 300 $\mu$L/well, and pat dry.

(9) Color development: Add TMB substrate solution, 100 $\mu$L/well, cover, and develop color in the dark at 15-25 °C for 2-15 min.

(10) Termination: Add stop solution (1 M H2SO4), 50 $\mu$L/well, to terminate the reaction.

(11) Detection: Measure the OD value of each well at a detection wavelength of 450 nm (reference wavelength 620 nm) using a microplate reader.

(12) Data processing and analysis: Use GraphPad Prism 8 software to fit the OD values of the standards obtained from the microplate reader to generate a standard curve equation and calculate the concentration results of each sample.

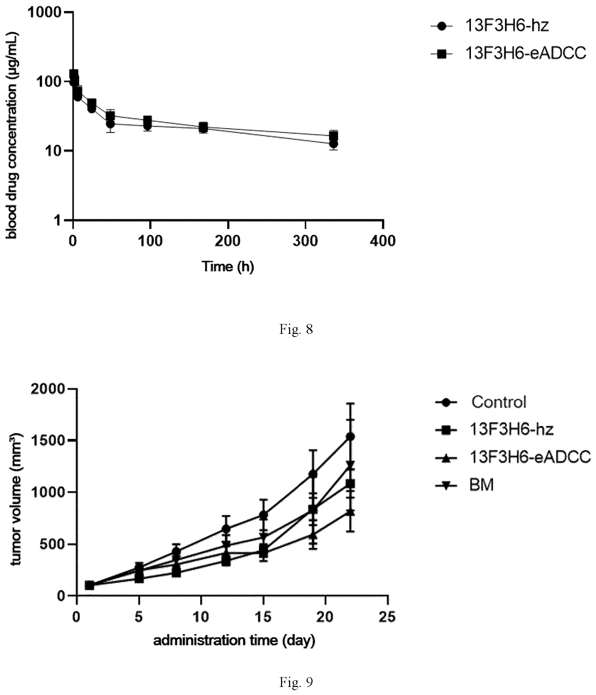

The experimental results are shown in Table 8 and Figure 8, indicating that the half-life of 13F3H6-hz and 13F3H6-eADCC in rats is approximately 12 days.

**Table 8. In Vivo Metabolism Study of Anti-CCR8 Humanized Antibody in Rats**

| Parameter | 13F3H6-hz | 13F3H6-eADCC |
|---|---|---|
| Cmax (ug·mL-1) | 116.61±8.05 | 130.37±16.74 |
| T1/2 (hr) | 294.07±120.14 | 282.13±7.90 |
| AUC-last (ug·h·mL-1) | 7887.43±641.21 | 9259.03±476.64 |
| AUC0-inf (ug·h·mL-1) | 13648.32±3974.18 | 16093.81±1750.27 |
| CL (mL·kg-1·h-1) | 0.7700±0.1925 | 0.6262±0.0672 |
| Vdss (mL·kg-1) | 281.36±48.04 | 241.03±10.85 |

**Example 18: In Vivo Efficacy Testing of Humanized Anti-Human CCR8 Antibody**

To verify the in vivo efficacy of humanized anti-human CCR8 antibodies, 6-8 week old female human CCR8 transgenic mice (C57BL/6J-Ccr8em3(hCCR8)/Smoc, purchased from Shanghai Model Organisms Center, Inc.) were used. MC38 cells (Sichuan Xpiscoric) in the logarithmic growth phase were digested with trypsin and resuspended in PBS. Each mouse was subcutaneously injected with $5 \times 10^6$ cells. Once the tumors reached a size of 100-200 mm$^3$, intravenous administration was performed twice a week at a dose of 10 mg/kg for a total of 6 doses. Tumor volume and mouse weight were measured throughout the experiment.
The main observation indicators for this experiment included:

1) Tumor Growth Inhibition (TGI) (%), calculated using the formula:

$$\text{TGI}\,(\%) = (1 - \text{T/C}) \times 100\%$$

where T and C represent the relative tumor volumes of the treatment group and control antibody BM at a specific time point, respectively.
2) Photographs of tumor volume at the endpoint and tumor weight.

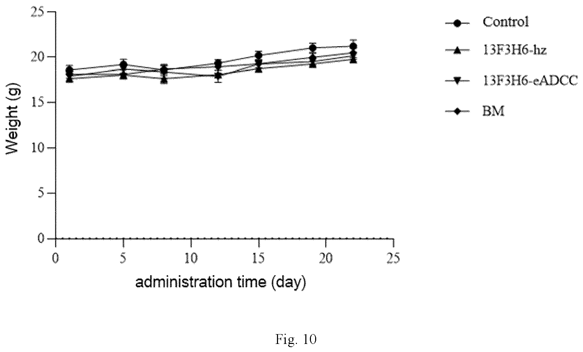

The experimental results are shown in Figure 9, indicating that both 13F3H6-hz and 13F3H6-eADCC inhibited MC38 tumor growth in mice, with TGI values of 29.5% and 47.0%, respectively, compared to a TGI of 18.2% for the control antibody. The changes in mouse weight are shown in Figure 10, demonstrating that the anti-CCR8 humanized antibody had no effect on mouse weight throughout the entire treatment process.

**Claims**

1.    An antibody that specifically binds to CCR8, wherein the antibody comprises a heavy chain variable region containing HCDR1, HCDR2, and HCDR3, wherein the HCDR1, HCDR2, and HCDR3 are selected from the corresponding amino acid sequences of any of the following combinations of HCDR1, HCDR2, and HCDR3:

(a) SEQ ID NO: 3, 4, and 5;
(b) SEQ ID NO: 6, 7, and 5;
(c) SEQ ID NO: 8, 9, and 5;
(d) SEQ ID NO: 10, 11, and 12;
(e) SEQ ID NO: 20, 21, and 22;
(f) SEQ ID NO: 23, 24, and 22;
(g) SEQ ID NO: 25, 26, and 22;
(h) SEQ ID NO: 27, 28, and 29;
(i) SEQ ID NO: 37, 38, and 39;
(j) SEQ ID NO: 40, 41, and 39;
(k) SEQ ID NO: 42, 43, and 39;
(l) SEQ ID NO: 44, 45, and 46;

and the antibody comprises a light chain variable region containing LCDR1, LCDR2, and LCDR3, wherein the LCDR1, LCDR2, and LCDR3 are selected from the corresponding amino acid sequences of any of the following combinations of LCDR1, LCDR2, and LCDR3:

(a) SEQ ID NO: 13, 14, and 15;
(b) SEQ ID NO: 16, 17, and 15;
(c) SEQ ID NO: 30, 31, and 32;
(d) SEQ ID NO: 33, 34, and 32;
(e) SEQ ID NO: 47, 48, and 49;
(f) SEQ ID NO: 50, 51, and 49.

2. The antibody according to claim 1, wherein the antibody comprises HCDR1, HCDR2, HCDR2, HCDR3, LCDR1, LCDR2, and LCDR3, which are selected from the corresponding amino acid sequences of any of the following combinations of HCDR1, HCDR2, HCDR2, HCDR3, LCDR1, LCDR2, and LCDR3:

(a) SEQ ID NO: 37, 38, 39, 47, 48, and 49;
(b) SEQ ID NO: 40, 41, 39, 47, 48, and 49;
(c) SEQ ID NO: 42, 43, 49, 47, 48, and 49;
(d) SEQ ID NO: 44, 45, 46, 50, 51, and 49;
(e) SEQ ID NO: 3, 4, 5, 13, 14, and 15;
(f) SEQ ID NO: 6, 7, 5, 13, 14, and 15;
(g) SEQ ID NO: 8, 9, 5, 13, 14, and 15;
(h) SEQ ID NO: 10, 11, 12, 16, 17, and 15;
(i) SEQ ID NO: 20, 21, 22, 30, 31, and 32;
(j) SEQ ID NO: 23, 24, 22, 30, 31, and 32;
(k) SEQ ID NO: 25, 26, 22, 30, 31, and 32;
(l) SEQ ID NO: 27, 28, 29, 33, 34, and 32.

3. The antibody according to claim 1, wherein the heavy chain variable region comprises the amino acid sequence shown in SEQ ID NO: 1, 18, or 35.

4. The antibody according to claim 1, wherein the light chain variable region comprises the amino acid sequence shown in SEQ ID NO: 2, 19, or 36.

5. The antibody according to claim 1, wherein the heavy chain variable region and the light chain variable region sequences are selected from any of the following combinations of amino acid sequences:

(a) The heavy chain variable region shown in SEQ ID NO: 1 and the light chain variable region shown in SEQ ID NO: 2;
(b) The heavy chain variable region shown in SEQ ID NO: 18 and the light chain variable region shown in SEQ ID NO: 19;
(c) The heavy chain variable region shown in SEQ ID NO: 35 and the light chain variable region shown in SEQ ID NO: 36.

6. The antibody according to claim 1, wherein the antibody's heavy chain variable region further comprises a human, humanized, or non-human framework region, and/or the antibody's light chain variable region further comprises a human, humanized, or non-human framework region.

7. The antibody according to claim 1, wherein the antibody is a monoclonal antibody, chimeric antibody, recombinant antibody, fully human antibody, humanized antibody, bispecific antibody, multispecific antibody, an antibody fragment, or an antigen-binding portion thereof.

8. The antibody according to claim 7, wherein the antibody is selected from the following combinations: (i) single-chain antibody or single-chain variable fragment, or a monovalent antibody lacking a hinge region; (ii) Fab, Fab', F(ab')$_2$; (iii) full-length antibody; (iv) an antibody comprising a human IgG Fc domain.

9. The antibody according to any one of claims 1-8, wherein the antibody exhibits one or more of the following biological activities:

(1) binds both cynomolgus monkey and human CCR8;
(2) has ADCC activity;
(3) has ADCP activity;
(4) does not bind human CCR4;
(5) has activity in blocking CCL1-mediated cell migration.

10. A recombinant protein comprising the antibody, antibody fragment, or antigen-binding portion thereof according to any one of claims 1-9.

11. A nucleic acid molecule, wherein the nucleic acid molecule encodes the antibody, antibody fragment, or antigen-binding portion thereof according to any one of claims 1-9, or the recombinant protein according to claim 10.

12. A vector comprising the nucleic acid molecule according to claim 11.

13. The vector according to claim 12, wherein the vector is selected from bacterial plasmids, bacteriophages, yeast plasmids, plant cell viruses, or mammalian cell viruses.

14. An engineered host cell comprising the nucleic acid molecule according to claim 11 or the vector according to claim 12.

15. An antibody-drug conjugate, wherein the conjugate comprises:

(i) a cytotoxic agent, detection label, drug, cytokine, radionuclide, enzyme, or a combination thereof; and
(ii) the antibody according to any one of claims 1-9, the recombinant protein according to claim 10, or a combination thereof.

16. A pharmaceutical composition, wherein the composition comprises:

(i) the antibody, antibody fragment, or antigen-binding portion thereof according to any one of claims 1-9, the recombinant protein according to claim 10, the nucleic acid molecule according to claim 11, the vector according to claim 12, the engineered host cell according to claim 14, the antibody-drug conjugate according to claim 15, or any combination thereof; and
(ii) a pharmaceutically acceptable carrier.

17. The pharmaceutical composition according to claim 16, wherein the pharmaceutical composition is used to inhibit the binding of CCR8 to any of its ligands, or as a neutralizing antibody.

18. The pharmaceutical composition according to claim 17, wherein it is used for depleting tumor-infiltrating Treg cells or for treating cancer.

19. A kit or delivery device comprising the antibody, antibody fragment, or antigen-binding portion thereof according to any one of claims 1-9, the recombinant protein according to claim 10, the nucleic acid molecule according to claim 11, the vector according to claim 12, the engineered host cell according to claim 14, the antibody-drug conjugate according to claim 15, or the pharmaceutical composition according to claim 16.

20. A method of producing a recombinant polypeptide, comprising:

(i) culturing the engineered host cell according to claim 14 under conditions suitable for expression; and
(ii) isolating the recombinant polypeptide from the culture, wherein the recombinant polypeptide is the antibody, antigen-binding portion, or recombinant protein according to any one of claims 1-10.

| | 96F1C4-hz | BM |
|---|---|---|
| EC50 | 79.64 | 99.45 |

Fig. 1A

| | 13F3H6-hz | 76H1F11-hz | BM |
|---|---|---|---|
| EC50 | 104.3 | 321.8 | 119.4 |

Fig. 1B

| | 13F3H6-hz | BM |
|---|---|---|
| EC50 | 220.7 | 4986 |

Fig. 2

Fig. 3A

Fig. 3B

| | 13F3H6-hz | 13F3H6-eADCC | BM |
|---|---|---|---|
| EC50 | 20.26 | 24.37 | 49.19 |

Fig. 4A

| | 96F1C4-hz | BM |
|---|---|---|
| EC50 | 5.206 | 10.79 |

Fig. 4B

| | BM | 76H1F11-hz | 13F3H6-hz | 13F3H6-eADCC |
|---|---|---|---|---|
| EC50 | 23.59 | 14.78 | 9.770 | 1.174 |

Fig. 4C

| | 96F1C4-hz | 13F3H6-hz | 13F3H6-eADCC | BM |
|---|---|---|---|---|
| EC50 | 107.3 | 112.1 | 64.68 | 75.08 |

Fig. 5

Fig. 6

| | Control | 37°C 7 Day | 37°C 14 Day |
|---|---|---|---|
| HillSlope | 1.647 | 1.363 | 1.247 |
| EC50 | 36.69 | 42.61 | 42.38 |

Fig. 7A

| | Control | 37°C 7 Day | 37°C 14 Day |
|---|---|---|---|
| HillSlope | 1.264 | 1.026 | 0.9943 |
| EC50 | 34.98 | 42.54 | 38.98 |

Fig. 7B

Fig. 8

Fig. 9

Fig. 10

**EP 4 552 647 A1**

## INTERNATIONAL SEARCH REPORT

| | International application No. |
|---|---|
| | **PCT/CN2023/105817** |

**A. CLASSIFICATION OF SUBJECT MATTER**

A61K39/395(2006.01)i; C12N15/13(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C12N, A61P

Documentation searched other than minimum documentation to the extent that such documents are included in the fields searched

Electronic data base consulted during the international search (name of data base and, where practicable, search terms used)

CNTXT, CNABS, VEN, ENTXT, 万方, WANFANG, CNKI, PubMed, GenBank, ISI web of knowledge: CCR8, 抗体, C-C chemokine receptor type 8, antibody, 四川思柏沃生物技术有限公司, SEQ ID NO: 1-57

**C. DOCUMENTS CONSIDERED TO BE RELEVANT**

| Category* | Citation of document, with indication, where appropriate, of the relevant passages | Relevant to claim No. |
|---|---|---|
| A | CN 113260381 A (SHIONOGI & CO., LTD. et al.) 13 August 2021 (2021-08-13)<br>claim 1 | 1-20 |
| A | CN 110835371 A (PUMISI BIO-TECH (SUZHOU) CO., LTD.) 25 February 2020 (2020-02-25)<br>entire document | 1-20 |
| A | WO 2007044756 A2 (ICOS CORPORATION) 19 April 2007 (2007-04-19)<br>entire document | 1-20 |
| A | WO 2021142002 A1 (VACCINEX, INC.) 15 July 2021 (2021-07-15)<br>entire document | 1-20 |
| A | WO 2022042690 A1 (HARBOUR BIOMED (SHANGHAI) CO., LTD.) 03 March 2022 (2022-03-03)<br>entire document | 1-20 |
| A | WO 2022078277 A1 (LANOVA MEDICINES LIMITED COMPANY) 21 April 2022 (2022-04-21)<br>entire document | 1-20 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 October 2023** | **25 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

EP 4 552 647 A1

**INTERNATIONAL SEARCH REPORT**

| International application No. |
| --- |
| **PCT/CN2023/105817** |

| Box No. I    Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

30

International application No.

**PCT/CN2023/105817**

| Patent document cited in search report | | | Publication date (day/month/year) | Patent family member(s) | | | Publication date (day/month/year) |
|---|---|---|---|---|---|---|---|
| CN | 113260381 | A | 13 August 2021 | WO | 2020138489 | A1 | 02 July 2020 |
| | | | | US | 2022064312 | A1 | 03 March 2022 |
| | | | | TW | 202039575 | A | 01 November 2020 |
| | | | | EP | 3903817 | A1 | 03 November 2021 |
| | | | | EP | 3903817 | A4 | 17 August 2022 |
| | | | | AU | 2019415395 | A1 | 15 July 2021 |
| | | | | JPWO | 2020138489 | A1 | 18 February 2021 |
| | | | | JP | 6894086 | B2 | 23 June 2021 |
| | | | | CA | 3124332 | A1 | 02 July 2020 |
| | | | | MX | 2021007576 | A | 24 August 2021 |
| | | | | SG | 11202106214 | YA | 29 July 2021 |
| | | | | JP | 2021101710 | A | 15 July 2021 |
| | | | | KR | 20210108996 | A | 03 September 2021 |
| CN | 110835371 | A | 25 February 2020 | None | | | |
| WO | 2007044756 | A2 | 19 April 2007 | WO | 2007044756 | A3 | 28 June 2007 |
| WO | 2021142002 | A1 | 15 July 2021 | IL | 294330 | A | 01 August 2022 |
| | | | | CA | 3160204 | A1 | 15 July 2021 |
| | | | | KR | 20220122628 | A | 02 September 2022 |
| | | | | US | 2021238292 | A1 | 05 August 2021 |
| | | | | BR | 112022011749 | A2 | 30 August 2022 |
| | | | | EP | 4087607 | A1 | 16 November 2022 |
| | | | | JP | 2023509323 | A | 08 March 2023 |
| | | | | TW | 202136314 | A | 01 October 2021 |
| | | | | AU | 2021205877 | A1 | 09 June 2022 |
| WO | 2022042690 | A1 | 03 March 2022 | JP | 2023538782 | A | 11 September 2023 |
| | | | | CA | 3190879 | A1 | 03 March 2022 |
| | | | | KR | 20230052910 | A | 20 April 2023 |
| | | | | EP | 4186926 | A1 | 31 May 2023 |
| | | | | TW | 202208439 | A | 01 March 2022 |
| | | | | AU | 2021330067 | A1 | 30 March 2023 |
| WO | 2022078277 | A1 | 21 April 2022 | CO | 2023004652 | A2 | 21 July 2023 |
| | | | | CA | 3198647 | A1 | 21 April 2022 |
| | | | | US | 2022195057 | A1 | 23 June 2022 |
| | | | | EP | 4010378 | A1 | 15 June 2022 |
| | | | | EP | 4010378 | A4 | 26 July 2023 |
| | | | | AU | 2021277712 | A1 | 05 May 2022 |
| | | | | AU | 2021277712 | B2 | 25 May 2023 |
| | | | | IL | 302078 | A | 01 June 2023 |
| | | | | KR | 20230088426 | A | 19 June 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113260381 A